# EUROPEAN PATENT APPLICATION

(11) **EP 3 900 624 A1**
(43) Date of publication of application: **27.10.2021**
(21) Application number: 20382317.4
(22) Date of filing: 20.04.2020
(51) Int. Cl.: A61B 5/0478

(54) **AN ELECTRODE DEVICE FOR AMPLITUDE-INTEGRATED ELECTROENCEPHALOGRAPHY**

(71) Applicant: Universitat Rovira I Virgili, 43003 Tarragona (ES); Institut d'Investigació Sanitària Pere Virgili, 43003 Tarragona (ES)
(72) Inventor: FABREGAT SANJUAN, Albert, 43007 Tarragona (ES); PASCUAL RUBIO, Vicenç, 43005 Tarragona (ES)
(74) Representative: Juncosa Miró, Jaime

(57) **Abstract**

An electrode device for aEEG is disclosed. The electrode device comprises a housing mounted on an adhesive element to adhere the electrode device to a skin surface of a patient; an electrode plate placed in the housing; and a wire connection for the electrode plate. The housing has a first protruding part that includes a conical-shaped hole for injecting and removing an electroconductive gel and that is disposed in the housing inclined outwardly at an angle between 50 degree and 70 degree with respect to the longitudinal direction of the skin surface. The housing also has a second protruding part that includes a hole for the introduction of the wire connection in order to be attached to the electrode plate. The second protruding part is disposed on the axis of the housing.

## Description

### Technical Field

The present invention is directed, in general, to the field of medical electrodes. In particular, the invention relates to an electrode device designed to record superficial brain bioelectric activity during long periods of time from infants in the intensive care units (ICU's). The electrode is particularly designed to be used in amplitude-integrated electroencephalography (aEEG), however it can also be used to record other long-term neurophysiological recording tests such as continuous electroencephalography monitoring (CEEG), among others.

### Background of the Invention

Amplitude-integrated electroencephalography (aEEG) is an easily accessible technique for monitoring the brain function in infants. In its simplest form, aEEG is a processed single-channel electroencephalogram that is filtered and time compressed.

There are several specific technical aspects for infant aEEG in the ICUs to be considered, beginning with the montage and electrode placement, the long-term recordings (several days), the lying position of the infants in the ICUs, the frequent manipulation by healthcare staff, the characteristic morphology of the head and the fragility of the scalp of preterm and term infants.

The known or commercial electrodes do not fulfill the requirements needed for infants.

Most available surface electrodes are not properly sized for term and preterm infant's application. Also, they are usually attached to a long wire that connects them to the bioelectric signal amplifier. This long wire electrode hinders the manipulation of the newborn by health care staff. In addition, the electrode wire is usually attached to the electrode laterally. In the lying patient, the usual position in the ICU's, this joint causes a lever force that facilitates the electrode decoupling.

Superficial cup electrodes available have a hole through which the electroconductive gel is replaced in order to maintain its high conductive capacity during long-term recordings. During this process, a blunt-tipped syringe is usually used to remove the old electroconductive gel and apply the new one. At this time, it is usually necessary to apply vertical pressure to beat the conductive gel layer that may have dried on the long-term recordings. This pressure can cause an involuntary contact with the scalp surface, which should be avoided in newborns due to their fragility and proximity to the fontanelles.

Disposable adhesive electrodes have the disadvantage of not allowing the replacement of the electroconductive gel and therefore they must be replaced periodically. This involves more manipulation and possible changes in the recording conditions (placement, impedance, etc.).

The subdermal needle electrodes which are often used in the aEEG to achieve low impedance recordings have the disadvantage of producing pain. Pain should always be avoided to improve comfort but in the aEEG it also has the disadvantage of not allowing the accurate assessment of a parameter as important as the latency of onset of the asleep-wake cycle. In addition, the subdermal electrodes have the same drawbacks as most surface electrodes available: are connected to a long wire to the amplifier and are easily detached.

There are known some patents and patents applications in this field.

FR2400370 discloses an electrode device intended to be applied to the skin, for electro-medical purposes. The electrode device can be used for electroencephalography, electrocardiography and electromyography. The electrode comprises a plastics body with a ring-shaped surface that is stuck to the skin by a double-sided tape. An electrode is arranged in a cavity that can be filled with a conductive paste or gel through a hole. The hole is arranged on the axis of the electrode body. At least one discharge duct from the cavity is provided for excess paste or gel, preferably in the form of a groove in the electrode body under the adhesive tape. This electrode has the disadvantage of having the long cable making it difficult for the healthcare staff to handle the child. The connection between the plate and the cable is lateral, facilitating the uncoupling of the electrode by lever forces in the lying patient. Moreover, this type of electrode device does not avoid the possible contact with the scalp during the replacement of electroconductive gel.

US 4657023-A provides a self-adhering electrode for application on the skin of a patient in which a portion of the electrically conductive layer forms an electrode that is substantially surrounded by a pressure-sensitive layer and the remainder of the conductive layer is covered by a substrate which is also conductive and sufficiently pliant to permit the electrode to adjust to the body contour. This electrode does not allow electrode conductive gel replacement without removing the electrode. It has a long wire laterally connected that complicates the manipulation of the infant and facilitates the uncoupling of the electrode by lever forces in the lying patient.

US 4029086-A provides an electrode arrangement which provides for a reliable electrical contact with the skin of a person or animal. The electrode arrangement comprises, in combination, a central post with a base flange, an adhesive pad having a central aperture for receiving the central post, a gel member and a resilient o-ring shaped member. An alternate embodiment comprises a central post and base flange, an adhesive pad having a central aperture for receiving the central post, and a shield washer, with a central aperture for receiving the central post, mounted above the adhesive pad. Both embodiments minimize the effect of physical forces placed on the electrical contact. This electrode has the same disadvantages as US 4657023-A

For these reasons, a new electrode device which solves the problems of current electrodes is needed.

### Description of the Invention

To that end the present invention proposes, according to one aspect, an electrode device for aEEG. As known in the field, the electrode device comprises a housing fixed on an adhesive element to adhere the electrode device to the skin surface of a patient, particularly the scalp; an electrode plate placed in the housing at a certain distance from a support surface of the housing forming a hollow space; and a wire connection for the electrode.

Unlike the known proposals in the field, in the proposed electrode device the housing has two protruding parts, a first protruding part and a second protruding part.

The first protruding part includes or provides a first hole of a conical shape for injecting and replacing an electroconductive gel inside the hollow space. The first protruding part is disposed in the housing inclined outwardly at an angle between 50-degree and 70-degree with respect to the longitudinal direction of the skin surface.

The second protruding part includes or provides a second hole for the introduction of the wire connection, so that the wire connection can be attached, e.g. by means of one or more welding points, to the electrode plate. The second protruding part is disposed, substantially, on the axis of the housing. Therefore, the wire connection is maintained perpendicular or almost perpendicular (about 90º) to the skin surface when performing the measures, which allows minimizing the tangential forces that favor the inclination of the electrode plate and it's uncoupling from the scalp. Likewise, the electrode plate is placed horizontally (i.e. flat or parallel, or substantially parallel, to the skin surface, e.g. the brain's cortex) in the housing. Hence, the sum of the postsynaptic potentials of the different pyramidal neurons oriented vertically to the brain cortex will be captured homogeneously by means of the whole surface of the electrode plate.

The proposed electrode device has a size and form adaptable to the head of the newborn. It is connected with a short wire that, once disconnected from an amplifier, facilitates the manipulation of the infant by the healthcare staff. The vertical orientation of the insertion between the electrode plate and the wire improves stability in the lying position. The conical hole prevents accidental contact of a blunt tip of a needle with the infant's scalp and allows the safe replacement of the electroconductive gel, thus facilitating the good signal quality during long-term recordings. Furthermore, by being a surface electrode, it does not cause discomfort to the patient.

In an embodiment, the electrode plate comprises a first gap which allows the electroconductive gel to flow through and a second gap to fix the rotating position of the electrode plate to the housing using a clamping element. Hence the distance between the skin surface and the electrode plate is reduced (i.e. the mentioned hollow space is smaller).

In a particular embodiment, the first protruding part is disposed at a 60-degree angle with respect to the longitudinal direction of the skin surface.

The first protruding part can include a plug for closure thereof. Hence the properties of the electroconductive gel can be kept for a longer period of time.

The housing can be made of a polymer, ceramic or metallic material. The electrode plate can be made of, or can comprise a coating made of, Silver (Ag), Gold (Au), Silver/Silver-Chloride (Ag/AgCI), or any good electrical conductor material (e.g. Tin, Stainless Steel, Platinum, Cooper, Lead, or Nichrome).

In an embodiment, the adhesive element is a flexible adhesive pad including an adhesive layer provided on a lower surface thereof. Alternatively, the adhesive element comprises a double-sided tape.

In an embodiment, the electrode device also has a connector such as a touchproof connector or a male snap connector, among others, arranged on a distal end of the wire connector.

In an embodiment, when the electrode device has to be used in newborns or infants, the length of the wire connection is less than 50 millimeters, particularly of approximately 30 millimeters. In other embodiments, for instance when the electrode has to be used in adults the length of the wire connection can be between 0.6 and 2 meters.

In some embodiments, the wire connection can also include an insulating layer, for example made of a material comprising silicone, Polyvinyl chloride, Polypropylene, Polyethylene, XLPE (Cross-Linked Polyethylene), EPR (Ethylene Propylene Rubber), ECTFE (ethylene chlorotrifluoroethylene), PVDF (Polyvinylidene fluoride), Nylon, CPE (Chlorinated polyethylene), etc.

In yet some embodiments, one or more sealing elements can be disposed between the housing and the insulating layer of the wire connection.

In an embodiment, the surface of the housing in contact with the skin or the adhesive element has a curvature that better fits with the skull shape.

Hence, the proposed electrode device allows an easy electrode placement as well as a wire cable extension that permits an easier electrical connection. The electrode device design also allows the patient, in particular newborns, to rotate the head without difficulties. The new design allows replacing the electroconductive gel for long-term aEEG recordings.

### Brief Description of the Drawings

The previous and other advantages and features will be more fully understood from the following detailed description of embodiments, with reference to the attached figures, which must be considered in an illustrative and non-limiting manner, in which:
Figs. 1A-1C illustrate different views of the proposed electrode device, according to an embodiment of the present invention.
Figs. 2A-2B illustrate different views of another embodiment of the proposed electrode device.
Fig. 3 illustrates another embodiment of the proposed electrode device. In this case, the surface of the housing configured to contact the skin or the adhesive element is curved. The adhesive element comprises a double-sided tape.
Figs. 4A-4B illustrate another embodiment of the proposed device in which the surface of the housing configured to contact the adhesive element is curved. In this case the adhesive element comprises a double-sided tape. Fig. 4B is an enlarged view of section B outlined in Fig. 4A.

### Detailed Description of Preferred Embodiments

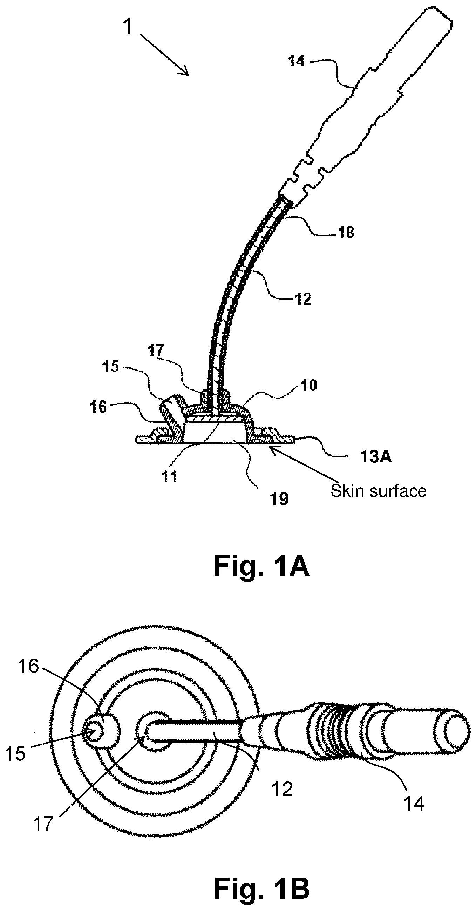

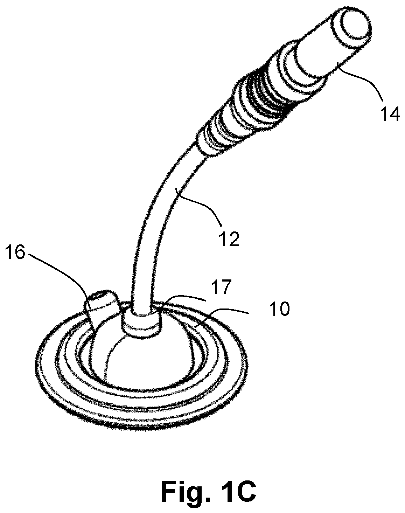

With reference to Figs. 1A-1C, therein it is showed a first embodiment of the proposed electrode device. The electrode device 1 of this embodiment includes a housing 10, for example made of a polymeric, ceramic or metallic material, which is mounted or arranged on a flexible adhesive pad 13A to adhere the electrode device 1 to a skin surface of a patient. The flexible adhesive pad 13A has an adhesive layer provided on a lower surface thereof to allow said adhesion of the electrode device 1 to the skin surface. As seen in the figures, the flexible adhesive pad 13A has a larger diameter than the housing 10.

The electrode device 1 also includes an electrode plate 11 horizontally disposed (i.e. parallel or substantially parallel to the skin surface of the patient) inside the housing 10 at a certain distance therein forming a hollow space 19. In particular, the electrode plate 11 is made of, or comprises a coating made of, Silver (Ag), Gold (Au), or Silver/Silver-Chloride (Ag/AgCI). In other embodiments however any other good electrical conductor material such as Tin, Stainless Steel, Platinum, Cooper, Lead, or Nichrome, among others, can be used.

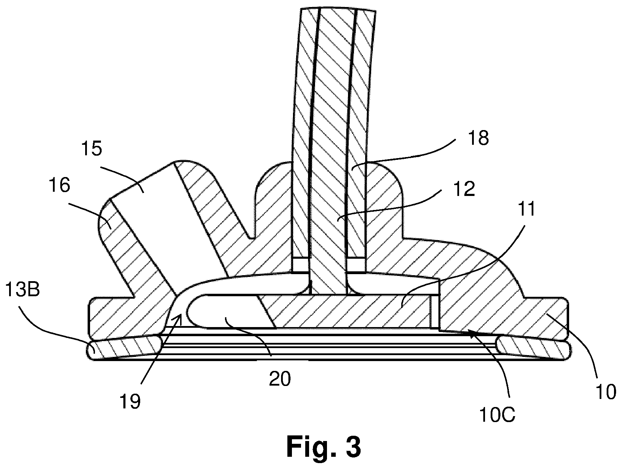

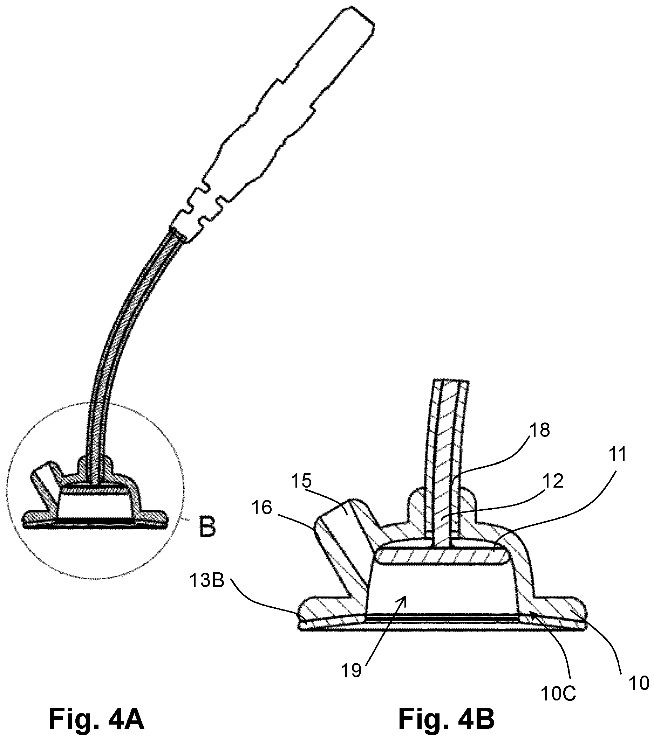

Also, the electrode device 1 includes a flexible wire connection 12 for the electrode plate 11. The wire connection 12 is fixed to the electrode plate by one of its extremes using at least one welding point, the other extreme being attached to a touchproof connector 14. It should be noted that in other embodiments, in this case not illustrated, the connector used can be of different type, for example a male snap connector, among others. Likewise, it should be noted that in other embodiments, an adhesive element comprising a double-sided tape 13B (see Fig. 3 and Figs. 4A and 4B for examples) can be used instead of the flexible adhesive pad 13A.

The housing 10 has two protruding parts 16, 17. In this particular embodiment, the first protruding part 16 is located inclined outwardly at an angle of approximately 60° with regard to the longitudinal direction of the skin surface. It should be noted that in other embodiments, in this case not illustrated, the first protruding part 16 can be located at an angle between 50º and 70º with respect to said longitudinal direction of the skin surface. The first protruding part 16 includes a conical-shaped hole 15 to allow the injection and removal of an electroconductive gel or hydrogel. The second protruding part 17 is located about the axis of the housing 10 and includes a second hole for the upright introduction of the wire connection 12.

Any biocompatible electroconductive gel can be used in accordance with the present disclosure. The electroconductive gel can be replaced via a blunt needle through the conical-shaped hole 15 but the needle is blocked by the geometry of the hole to avoid skin injuries.

Particularly, the electrode device 1 of Figs. 1A-1C also includes an insulting layer 18 surrounding the wire connection. Different materials can be used as insulating layer for example silicone, Polyvinyl chloride, Polyethylene, Polyvinylidene fluoride and/or Polypropylene, among others.

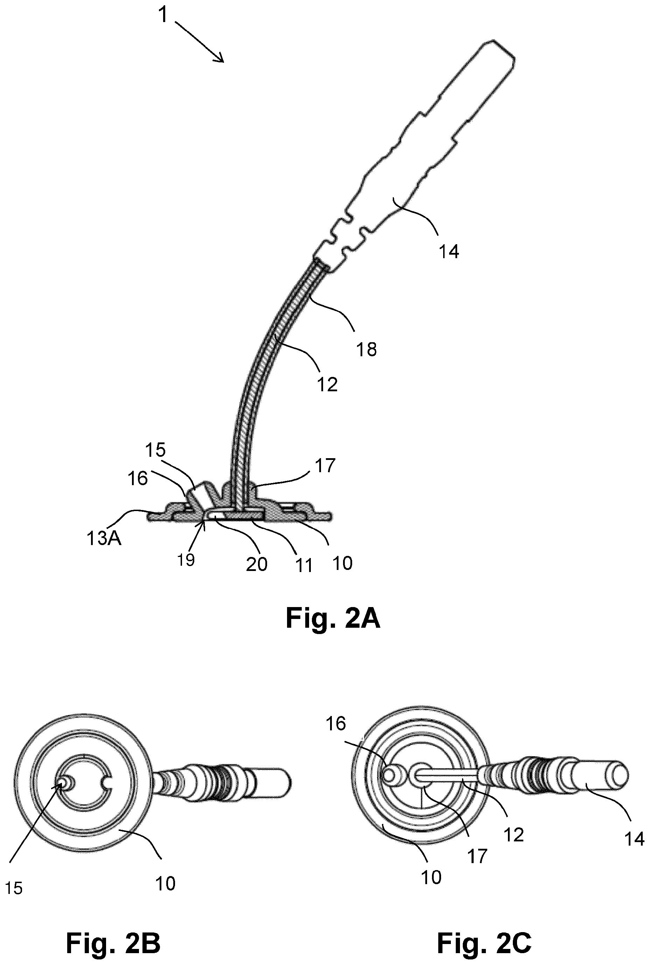

With regard to Figs. 2A-2C, therein another embodiment of the proposed electrode device 1 is illustrated. In this case, different to the embodiment of Figs. 1A-1C, the hollow space 19 is reduced since the electrode plate 11 is pierced in two parts: one opening 20 is disposed to fit with the conical-shaped hole 15 to allow the electroconductive gel to flow through; the other opening (not seen in the figures) is used for the fixation of the electrode plate 11 to the housing 10, for example via a clamping or fixing element.

Fig. 3 illustrates an embodiment in which the surface 10C of the housing 10 that has to contact the adhesive element comprises a curvature to better fit with the skull. The size of the housing 10 in this case is reduced. In this embodiment, particularly, the adhesive element comprises a double-sided tape 13B.

Figs. 4A and 4B illustrate another embodiment in which the surface 10C of the housing 10 is curved. In this embodiment, the adhesive element comprises a double-sided tape 13B and the electrode plate 11 is not pierced.

In some embodiments, sealing elements can be also disposed between the housing 10 and the insulating layer 18 of the wire connection 12. In some embodiments also, the first protruding part 16 can comprise a plug for closure thereof.

It will be understood that various details of the presently disclosed matter may be changed without departing from the scope of the presently disclosed matter. Furthermore, the previous description is for the purpose of illustration only, and not for the purpose of limitation.

As used herein, the term "about" and/or "substantially", when referring to a value or to feature, are meant to encompass variations of in some embodiment's ±10%, in some embodiment's ±5%, from the specified value or feature, as such variations are appropriate to perform the disclosed device.

The scope of the present invention is defined in the following set of claims.

## Claims

1. An electrode device for amplitude-integrated electroencephalography (aEEG), comprising:
a housing (10) mounted on an adhesive element (13A, 13B) to adhere the electrode device (1) to a skin surface of a patient, the housing (10) having a first hole (15);
an electrode plate (11) placed in the housing (10) at a certain distance from a support surface of the housing (11) forming a hollow space (19);
a wire connection (12) for the electrode plate (11), said wire connection (12) having a given length and comprising a proximal end and a distal end;
the first hole (15) being for injecting an electroconductive gel inside the hollow space (19),
**characterized in that** the housing (10) comprises:
a first protruding part (16) that includes the first hole (15), the latter having a conical-shaped geometry, the first protruding part (16) being disposed in the housing (10) inclined outwardly at an angle between 50-degree and 70-degree with respect to the longitudinal direction of the skin surface; and
a second protruding part (17) that includes a second hole through which the proximal end of the wire connection (12) is introduced and attached to the electrode plate (11), the second protruding part (17) being disposed on the axis of the housing (10).

2. The electrode device of claim 1, wherein the first protruding part (16) is disposed at a 60-degree angle with respect to the longitudinal direction of the skin surface.

3. The electrode device of any of previous claims, wherein the wire connection (12) further comprises a connector (14) on said distal end.

4. The electrode device of any of previous claims, wherein the adhesive element is a flexible adhesive pad (13A) comprising an adhesive layer provided on a lower surface thereof.

5. The electrode device of any of previous claims 1 to 3, wherein the adhesive element is a double-sided tape (13B).

6. The electrode device of any of previous claims, wherein the attachment of the wire connection (12) to the electrode plate (11) comprises one or more welding points.

7. The electrode device of any of previous claims, wherein the wire connection (12) further comprises an insulating layer (18).

8. The electrode device of claim 7, wherein the insulating layer is made of a material comprising silicone, Polyvinyl chloride, Polyethylene, Polyvinylidene fluoride and/or Polypropylene.

9. The electrode device of claim 7 or 8, further comprising one or more sealing elements disposed between the housing (10) and the insulating layer (18) of the wire connection (12).

10. The electrode device of any of previous claims, wherein the length of the wire connection (12) is less than 50 millimeters.

11. The electrode device of any of previous claims 1 to 9, wherein the length of the wire connection (12) is between 0.6 and 2 meters.

12. The electrode device of any of previous claims, wherein:
the housing (10) is made of a polymer, ceramic or metallic material; and
the electrode plate (11) is made of, or comprises a coating made of, Silver (Ag), Gold (Au), Silver/Silver-Chloride (Ag/AgCI), Tin, Stainless Steel, Platinum, Cooper, Lead, or Nichrome.

13. The electrode device of any of previous claims, wherein the first protruding part (16) further comprises a plug for closure thereof.

14. The electrode device of any of previous claims, wherein the electrode plate (11) comprises a first gap adapted to allow the electroconductive gel to flow through and a second gap adapted to fix a rotating position of the electrode plate (11) to the housing (10) using a clamping element.

15. The electrode device of any of previous claims, wherein a surface (10C) of the housing (10) configured to receive contact with the skin surface of the patient or the adhesive element (13A, 13B) has a given curvature.
